(19) Europäisches Patentamt
European Patent Office
Office européen des brevets

(11) Veröffentlichungsnummer: **0 127 062**
**A2**

(12) # EUROPÄISCHE PATENTANMELDUNG

(21) Anmeldenummer: 84105495.0

(22) Anmeldetag: 15.05.84

(51) Int. Cl.³: C 07 D 417/12
C 07 D 413/12, C 07 D 403/12
C 07 D 401/12, A 01 N 47/44

(30) Priorität: 28.05.83 DE 3319455

(43) Veröffentlichungstag der Anmeldung: 05.12.84 Patentblatt 84/49

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Schmitt, Hans-Georg, Dr.
Gustav-Freytag-Strasse 2
D-5090 Leverkusen 1(DE)

(72) Erfinder: Schade, Gerold, Dr.
Hahnenweg 8
D-5000 Köln 80(DE)

(72) Erfinder: Oeckl, Siegfried, Dr.
Auf der Hoehe 74
D-5060 Bergisch-Gladbach 2(DE)

(72) Erfinder: Brandes, Wilhelm, Dr.
Eichendorffstrasse 3
D-5653 Leichlingen(DE)

(54) **Alkylen-bis-heterocyclyl-biguanide.**

(57) Neue Alkylen-bis-heterocyclyl-biguanide der allgemeinen Formel (I)

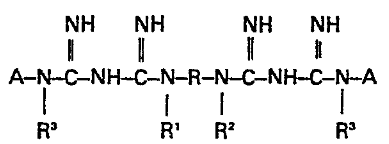

(I)

in welcher

A für gegebenenfalls substituierte Heterocyclen steht,

R für Alkylen steht und

$R^1$, $R^2$ und $R^3$ gleich oder verschieden sind und für Wasserstoff, Alkyl oder gegebenenfalls substituiertes Aralkyl stehen,

und deren Säureadditionssalze

sowie ihre Verwendung als Schädlingsbekämpfungsmittel, insbesondere als Fungizide.

Die neuen Alkylen-bis-heterocyclyl-biguanide können hergestellt werden, indem man geeignete Alkylen-bis-dicyandiamide mit geeigneten Aminoheterocyclen umsetzt.

EP 0 127 062 A2

Croydon Printing Company Ltd.

0127062



BAYER AKTIENGESELLSCHAFT 5090 Leverkusen, Bayerwerk

Konzernverwaltung RP
Patentabteilung

Bas/by-c
Ia

## Alkylen-bis-heterocyclyl-biguanide

Die vorliegende Erfindung betrifft neue Alkylen-bis-heterocyclyl-biguanide, ein Verfahren zu ihrer Herstellung und ihre Verwendung als Schädlingsbekämpfungsmittel.

Es ist bereits bekannt geworden, daß Alkylen-bis-arylbiguanide, wie z.B. das Chlorhexidin der Formel

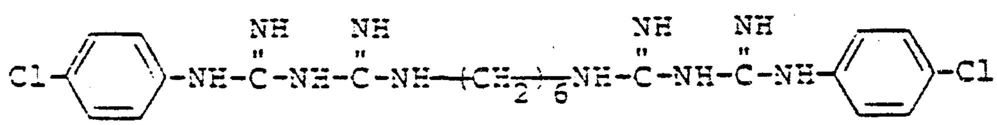

im technischen Bereich und im Humanbereich zu Desinfektionszwecken eingesetzt werden (vgl. Ullmann's Encyclopädie der technischen Chemie, 4. Auflage, Band 10, Seite 54, Verlag Chemie Weinheim 1975). Über eine Wirkung gegen Schädlinge im Pflanzenschutz ist nichts bekannt.

Weiterhin ist seit langem bekannt, daß N-sulfenylierte Dicarbonsäureimide, wie das N-Trichlormethylthio-tetrahydrophthalimid, eine fungizide Wirksamkeit besitzen (vgl. DBP 1 193 498).

Es wurden neue Alkylen-bis-heterocyclyl-biguanide der allgemeinen Formel (I) gefunden

$$A-\underset{R^3}{N}-\overset{NH}{\overset{\|}{C}}-NH-\overset{NH}{\overset{\|}{C}}-\underset{R^1}{N}-R-\underset{R^2}{N}-\overset{NH}{\overset{\|}{C}}-NH-\overset{NH}{\overset{\|}{C}}-\underset{R^3}{N}-A \qquad (I)$$

in welcher

A für gegebenenfalls substituierte Heterocyclen steht,

R für Alkylen steht und

$R^1$, $R^2$ und $R^3$ gleich oder verschieden sind und für Wasserstoff, Alkyl oder gegebenenfalls substituiertes Aralkyl stehen und

deren Säureadditionssalze.

Weiterhin wurde gefunden, daß man die Alkylen-bis-heterocyclyl-biguanide der allgemeinen Formel (I)

$$A-\underset{R^3}{N}-\overset{NH}{\overset{\|}{C}}-NH-\overset{NH}{\overset{\|}{C}}-\underset{R^1}{N}-R-\underset{R^2}{N}-\overset{NH}{\overset{\|}{C}}-NH-\overset{NH}{\overset{\|}{C}}-\underset{R^3}{N}-A \qquad (I)$$

in welcher

A für gegebenenfalls substituierte Heterocyclen steht,

R für Alkylen steht und

$R^1$, $R^2$ und $R^3$ gleich oder verschieden sind und für Wasserstoff, Alkyl oder gegebenenfalls substituiertes Aralkyl stehen,

erhält, wenn man

Alkylen-bis-dicyandiamide der allgemeinen Formel (II)

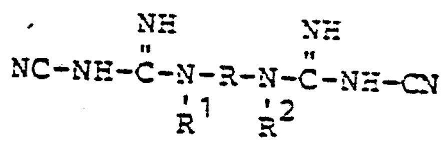

(II)

in welcher
R, $R^1$ und $R^2$ die oben angegebene Bedeutung haben,

mit Amino-heterocyclen der allgemeinen Formel (III)

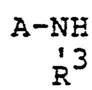

(III)

in welcher
$R^3$ und A die oben angegebene Bedeutung haben,

als freie Verbindungen oder in Salzform, gegebenenfalls in einem Lösungs- oder Verdünnungsmittel bei Temperaturen zwischen 80 und 200°C umsetzt.

Die neuen Alkylen-bis-heterocyclyl-biguanide und deren Säureadditionssalze weisen starke fungizide Eigenschaften auf. Die erfindungsgemäßen Stoffe stellen somit eine Bereicherung der Technik dar.

Die erfindungsgemäßen Alkylen-bis-heterocyclyl-biguanide sind durch die allgemeine Formel (I) allgemein definiert. In dieser Formel stehen vorzugsweise

A    für einen gegebenenfalls einfach bis sechsfach, gleich oder verschieden substituierten 5- oder 6-gliedrigen, gesättigten oder gegebenenfalls teilweise oder vollständig ungesättigten Heterocyclus mit 1 bis 4 gleichen oder verschiedenen Heteroatomen.

Der Heterocyclus kann gegebenenfalls benzoannelliert sein und/oder ein oder mehrere Ketogruppen im Heterocyclus enthalten. Als Substituenten der Heterocyclen kommen in Frage: Halogen, Alkyl mit 1 bis 18 Kohlenstoffatomen, Alkylthio, Alkoxy, Alkylsulfonyl mit jeweils 1 bis 8 Kohlenstoffatomen im Alkylteil und gegebenenfalls ein- bis sechsfach, gleich oder verschieden durch Halogen, Alkyl mit 1 bis 4 Kohlenstoffatomen und/oder Halogenalkyl mit 1 bis 3 Kohlenstoffatomen und 1 bis 5 Halogenatomen substituiertes Phenyl, ferner Halogenalkoxy, Halogenalkylthio und/oder Halogenalkylsulfonyl mit 1 bis 3 Kohlenstoffatomen und 1 bis 5 gleichen oder verschiedenen Halogenatomen im jeweiligen Halogenalkyl-Rest. Weitere Substituenten können sein Phenoxy, Phenylthio und/oder Phenylsulfonyl, wobei die jeweiligen Phenylreste gegebenenfalls substituiert sind

durch Halogen und/oder Alkyl mit 1 bis 4 Kohlenstoffatomen. Der ankondensierte Benzolring kann gegebenenfalls ein- oder zweifach, gleich oder verschieden durch Halogen, Alkyl, Alkoxy und Alkylthio mit jeweils 1 bis 4 Kohlenstoffatomen in dem jeweiligen Alkylteil, substituiert sein.

R für geradkettiges oder verzweigtes Alkylen mit 2 bis 10 Kohlenstoffatomen und

$R^1$, $R^2$ und $R^3$ gleich oder verschieden sind und für Wasserstoff, Alkyl mit 1 bis 20 Kohlenstoffatomen oder für Aralkyl mit 6 bis 10 Kohlenstoffatomen im Aryl- und 1 bis 6 Kohlenstoffatomen im Alkylteil, wobei der Arylteil ein- bis fünffach, gleich oder verschieden durch Alkyl mit 1 bis 6 Kohlenstoffatomen und/oder Halogen substituiert sein kann.

Besonders bevorzugt sind Verbindungen der allgemeinen Formel (I), in denen

A für einen gegebenenfalls ein- bis vierfach, gleich oder verschieden substituierten 5- oder 6-gliedrigen gesättigten, teilweise oder vollständig ungesättigten Heterocyclus mit 1 oder 2 Sauerstoff- und/oder Schwefel- und/oder 1 bis 4 Stickstoffatomen steht, der gegebenenfalls benzoannelliert

ist und gegebenenfalls eine oder zwei Ketogruppen enthält. Als Substituenten der Heterocyclen kommen in Frage: Fluor, Chlor und Brom, Alkyl, Alkylthio, Alkoxy und Alkylsulfonyl mit jeweils 1 bis 6 Kohlenstoffatomen im Alkylteil, wie Methyl, Ethyl, n-Propyl, iso-Propyl, n-Butyl, iso-Butyl, sec.-Butyl, tert.-Butyl, Pentyl, Hexyl, Methylthio, Ethylthio, n-Propylthio, iso-Propylthio, n-Butylthio, iso-Butylthio, sec.-Butylthio, tert.-Butylthio, Pentylthio, Hexylthio, Methoxy, Ethoxy, n-Propoxy, iso-Propoxy, n-Butoxy, iso-Butoxy, sec.-Butoxy, tert.-Butoxy, Pentoxy, Hexoxy, Methylsulfonyl, Ethylsulfonyl, n-Propylsulfonyl, iso-Propylsulfonyl, n-Butylsulfonyl, iso-Butylsulfonyl, sec.-Butylsulfonyl, tert.-Butylsulfonyl, Pentylsulfonyl, Hexylsulfonyl, ferner gegebenenfalls ein- bis vierfach, gleich oder verschieden durch Fluor, Chlor, Brom, Methyl, Ethyl, n-Propyl, iso-Propyl, Trichlormethyl, Trifluormethyl, Dichlorfluormethyl und Tetrachlorethyl substituiertes Phenyl, ferner Halogenalkoxy, Halogenalkylthio und Halogenalkylsulfonyl mit 1 oder 2 Kohlenstoffatomen und 1 bis 5 Chlor- und/oder Fluoratomen, wie Trichlormethoxy, Trifluormethoxy, Dichlorfluormethoxy, 1,2,2-Trichlorethyl, Trifluormethylthio, Trichlormethylthio, Dichlorfluormethylthio, 1,2,2-Trichlorethylthio, Trichlormethylsulfonyl, Trifluormethylsulfonyl, Dichlorfluormethylsulfonyl, 1,2,2-Trichlorethylsulfonyl, ferner gegebenenfalls ein-bis dreifach, gleich oder verschieden durch Fluor, Chlor, Brom,

Methyl, Ethyl, n-Propyl und iso-Propyl substituiertes Phenoxy, Phenylthio oder Phenylsulfonyl. Der ankondensierte Benzolring kann gegebenenfalls ein- bis dreifach, gleich oder verschieden durch Chlor, Fluor, Methyl, Ethyl, n-Propyl, iso-Propyl, Methoxy, Ethoxy, n-Propoxy, iso-Propoxy, Methylthio, Ethylthio, n-Propylthio und/oder iso-Propylthio substituiert sein.

R für geradkettiges oder verzweigtes Alkylen mit 2 bis 8 Kohlenstoffatomen, wie Methylen, Ethylen, iso-Propylen, n-Propylen, n-Butylen, iso-Butylen, sec.-Butylen, Tetramethylen, Pentamethylen, Hexamethylen, Heptamethylen, Octamethylen, 2,5-Dimethylhexylen-, 1-Methylethylen, steht und

$R^1$,$R^2$ und $R^3$ gleich oder verschieden sind und für Wasserstoff, Alkyl mit 1 bis 10 Kohlenstoffatomen, wie Methyl, Ethyl, n-Propyl, iso-Propyl, n-Butyl, sec.-Butyl, iso-Butyl, tert.-Butyl, Pentyl, Hexyl, Heptyl, Octyl, Nonyl oder Decyl, oder für Phenethyl oder Benzyl, steht, wobei der jeweilige Phenylrest ein- bis dreifach, gleich oder verschieden durch Alkyl mit 1 bis 4 Kohlenstoffatomen, wie Methyl, Ethyl, n-Propyl, iso-Propyl, n-Butyl, sec.-Butyl, iso-Butyl und tert.-Butyl, und Halogen, wie Fluor, Chlor, Brom und Jod, substituiert sein kann.

Ganz besonders bevorzugt sind Verbindungen der allgemeinen Formel (I), in denen

A für einen gegebenenfalls ein- bis dreifach, gleich oder verschieden substituierten Thienyl-, Benzothienyl-, Furyl-, Pyrrolyl-, Imidazolyl-, Pyrazolyl-, Pyridyl-, Pyrazinyl-, Pyrimidinyl-, Pyridazinyl-, Chinolyl-, Phthalazinyl-, Chinoxalinyl-, Isoxazolyl-, Isothiazolyl-, Furazanyl-, Pyrrolidinyl-, Imidazolidinyl-, 1,2,3-Triazolidinyl-, 1,2,4-Triazolyl-, 1,2,3-Triazolyl-, 1,3,4-Triazolyl-, Tetrazolyl-, Thiazolyl-, Oxazolyl-, 1,2,4-Thiadiazolyl-, 1,2,5-Thiadiazolyl-, Benzthiazolyl-, Benzoxazolyl- oder Benzimidazolylrest steht, der gegebenenfalls eine Ketogruppe enthalten kann. Als Substituenten der Heterocyclen kommen in Frage: Chlor, Fluor, Methyl, Ethyl, iso-Propyl, Methylthio, Ethylthio, Methoxy, Ethoxy, Trifluormethyl, Trifluormethylthio, Phenyl, Chlorphenyl, Trifluormethyl-phenyl, Trifluormethylthio-phenyl, Phenoxy. Als Substituenten für den ankondensierten Benzolring seien genannt: Chlor, Fluor, Methyl, Ethyl, Methoxy und Methylthio,

R steht für Alkylen mit 6 Kohlenstoffatomen,

$R^1$ und $R^2$ für Wasserstoff und

$R^3$ für Wasserstoff oder Methyl stehen.

Verwendet man beispielsweise für das erfindungsgemäße Verfahren Hexamethylen-bis-dicyan-diamid und 3-Amino-1,2,4-triazol als Ausgangsverbindungen, so kann der

Reaktionsverlauf durch das folgende Formelschema wiedergegeben werden:

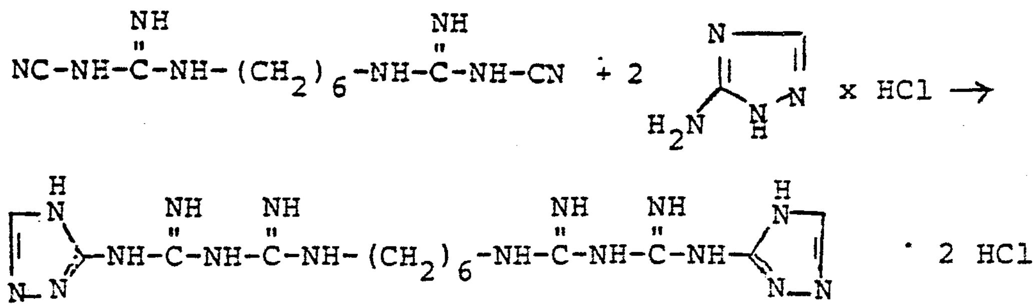

Die bei dem erfindungsgemäßen Verfahren als Ausgangsstoffe einzusetzenden Alkylen-bis-dicyandiamide der Formel (II) sind größtenteils bekannt, die bekannten ebenso die gegebenenfalls noch neuen Verbindungen sind nach bekannten Verfahren herstellbar (vgl. Britische Patente 631.878 und 702.268).

Die bei der Durchführung des erfindungsgemäßen Verfahrens als Ausgangsstoffe zu verwendenden Aminoheterocyclen der Formel (III) bzw. deren Säureadditionssalze sind bekannt und nach Analogieverfahren herstellbar.

Für die erfindungsgemäße Umsetzung kommen als Verdünnungs- oder Lösungsmittel organische Lösungsmittel in Frage. Hierzu gehören vorzugsweise Alkohole, wie Methanol, Ethanol, iso-Propanol, n-Propanol, n-Butanol, iso-Butanol, Amylalkohol, Cyclohexanol, Phenol; Glykole und Glykolether, wie Ethylenglykol, Ethylenglykolmono- und dialkylether, Diethylenglykol; sowie Kohlenwasserstoffe und Halogenkohlenwasserstoffe,

wie Toluol, Chloraromaten, Paraffinöl, Carbonsäuren, wie Ameisensäure, Essigsäure und Propionsäure, außerdem auch Wasser.

Bevorzugt wird ohne Lösungsmittel oberhalb des Schmelzpunktes des Reaktionsgemisches gearbeitet.

Die Reaktion kann bei Normaldruck oder auch bei erhöhtem Druck durchgeführt werden.

Die Reaktionstemperaturen können bei der Durchführung des erfindungsgemäßen Verfahrens in einem größeren Bereich variiert werden. Im allgemeinen arbeitet man zwischen 80°C und 200°C, vorzugsweise zwischen 100°C und 170°C.

Der Verlauf der Reaktion ist durch Abnahme der CN-Bande im IR-Spektrum gut zu verfolgen, das Reaktionsende zeigt sich demnach durch Verschwinden der CN-Bande; es kann jedoch selbstverständlich auch früher abgebrochen werden, wo z.B. die thermische Stabilität der Einsatz- oder Endprodukte nicht ausreicht, um einen 100 %igen Umsatz ohne Ausbeuteverlust zu erzielen.

Die Aufarbeitung und Reinigung erfolgt durch übliche Methoden, wie z.B. Zerkleinern, Auflösen, Filtrieren, Eindampfen, Umkristallisieren.

In einer bevorzugten Ausführungsform werden die Heterocyclen in Salzform eingesetzt.

Es können sowohl die Salze organischer wie auch anorganischer Säuren verwendet werden, wie z.B. der Ameisensäure, Essigsäure, Salzsäure, Schwefelsäure, Phosphorsäure. Bevorzugt werden die Hydrochloride eingesetzt. Dabei kann auch die Salzbildung in einem unmittelbar der Reaktion vorausgehenden Verfahrensschritt durchgeführt werden.

Im allgemeinen geht man folgendermaßen vor:

0,2 Mol des Heterocyclus und 0,1 Mol des Alkylen-bisdicyandiamids werden innig gemischt und unter Rühren auf 140°C bis 160°C erhitzt, wobei im Temperaturbereich 120-140°C langsam erhitzt werden muß, um die in einigen Fällen einsetzende stark exotherme Additionsreaktion unter Kontrolle zu halten. Die resultierenden Schmelzen werden gerührt, bis eine Probe im IR-Spektrum keine Cyanobande mehr zeigt. Nach dem Abkühlen werden glasige Produkte erhalten, die gepulvert werden können. Sie erweichen zum Teil beim Erwärmen ohne scharfen Schmelzpunkt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

So können z.B. fungizide Mittel im Pflanzenschutz eingesetzt werden zur Bekämpfung von Plasmodiophoromycetes,

Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Im Pflanzenschutz können die erfindungsgemäßen Wirkstoffe mit besonders gutem Erfolg zur Bekämpfung von Botrytis Arten, gegen pilzliche Krankheitserreger am Reis wie gegen Pellicularia und Pyricularia eingesetzt werden. Die Verbindungen zeigen auch eine akarizide Wirkung bei entsprechender Anwendung.

Die Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnapthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmiteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine, wie Calcit, Marmor, Bims, Sepiolith, Dolomit sowie synthetische Granulate aus anorganischen und organischen Mehlen sowie Granulate aus anorganischem Material, wie Sägemehl,

Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Herstellungsbeispiele

Beispiel 1

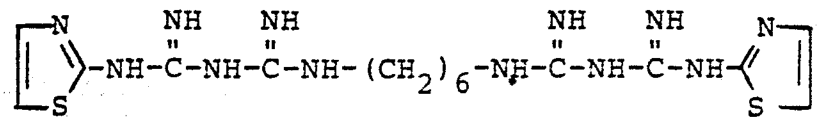 x2HCl

29,4 g (0,2 Mol) 93,2 %iges 2-Aminothiazol-hydrochlorid werden zusammen mit 25,0 g (0,1 Mol) 1,6-Bis-(cyanguanidino)-hexan fein gepulvert und in einen 250 ml Dreihalskolben mit Thermometer und mechanischem Rührer übergeführt. Beim Erwärmen auf 60°C wird das Gemisch dünnflüssig und verfärbt sich langsan nach mittelbraun. Die Temperatur wird allmählich weiter gesteigert und bei 120°C setzt eine exotherme Reaktion ein, wobei der Kolbeninhalt zähflüssig wird. Es wird zunächst 6 Stunden bei 140°C, dann weitere 6 Stunden bei 160°C gerührt. Nach dieser Zeit ist im IR-Spektrum die Cyanbande nicht mehr nachweisbar. Der Kolbeninhalt wird auf -70°C gekühlt und die dann spröde Masse pulverisiert.

Man erhält 46 g an Dihydrochlorid des 1,6-Bis-(2-thiazolylbiguanidino)-hexans als graues Pulver, das unter Zersetzung bei 210°C schmilzt.

Analog können die Verbindungen der allgemeinen Formel (I) hergestellt werden:

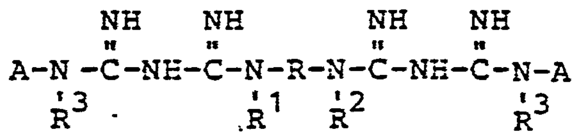 (I)

| Bsp. Nr. | R | $R^1$ | $R^2$ | $R^3$ | A | | Physikalische Konstanten: Schmelzpunkt (°C) |
|---|---|---|---|---|---|---|---|
| 2 | $(-CH_2-)_6$ | H | H | H | 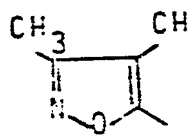 | x 2 HCl | 110-115 |
| 3 | $(-CH_2-)_6$ | H | H | H | 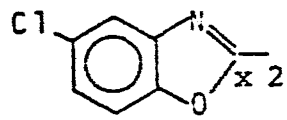 | x 2 HCl | 112-116 |
| 4 | $(-CH_2-)_6$ | H | H | H | 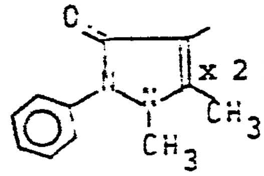 | x 2 HCl | 147-153 |
| 5 | $(-CH_2-)_6$ | H | H | H | 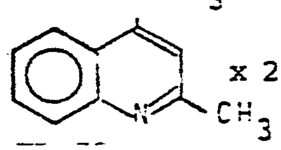 | x 2 HCl | 260-270 |
| 6 | $(-CH_2-)_6$ | H | H | H | 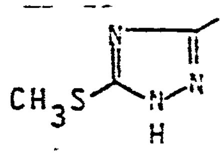 | x 2 HCl | 70-75 |
| 7 | $(-CH_2-)_6$ | H | H | H | 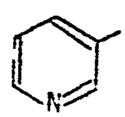 | x 2 HCl | 98-103 |
| 8 | $(-CH_2-)_6$ | H | H | H | 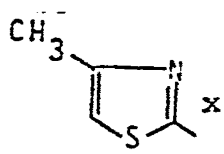 | x 2 HCl | 75-80 |

| Bsp. Nr. | R | $R^1$ | $R^2$ | $R^3$ | A | Physikalische Konstanten: Schmelzpunkt (°C) |
|---|---|---|---|---|---|---|
| 9 | $(-CH_2-)_6$ | H | H | H | 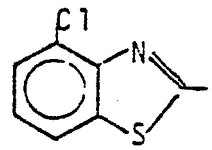 x 2 HCl | 150-155 |
| 10 | $(-CH_2-)_6$ | H | H | H | 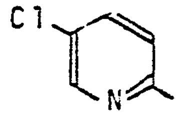 x 2 HCl | 130-135 |
| 11 | $(-CH_2-)_6$ | H | H | H | 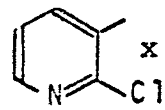 x 2 HCl | 59-65 |
| 12 | $(-CH_2-)_6$ | H | H | H | 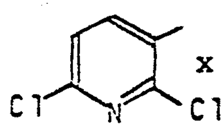 x 2 HCl | 93-97 |
| 13 | $(-CH_2-)_6$ | H | H | H | 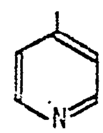 x 2 HCl | 130-135 |
| 14 | $(-CH_2-)_6$ | H | H | H | 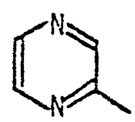 x 2 HCl | 160-170 |
| 15 | $(-CH_2-)_6$ | H | H | H | 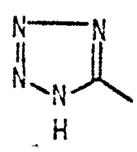 x 2 HCl | 153-157 |
| 16 | $(-CH_2-)_6$ | H | H | H | 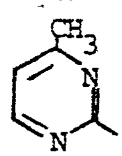 x 2 HCl | 270 (Z) |

| Bsp. Nr. | R | $R^1$ | $R^2$ | $R^3$ | A | Physikalische Konstanten: Schmelzpunkt (°C) |
|---|---|---|---|---|---|---|
| 17 | $(-CH_2-)_6$ | H | H | H | 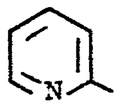 x 2 HCl | 140-142 (Z) |
| 18 | $(-CH_2-)_6$ | H | H | H | 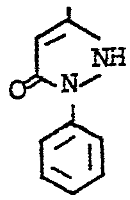 x 2 HCl | 235 (Z) |
| 19 | $(-CH_2-)_6$ | H | H | H | 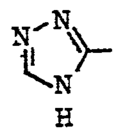 x 2 HCl | 155 (Z) |
| 20 | $(-CH_2-)_6$ | H | H | H | 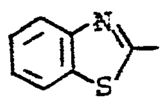 x 2 HCl | 200 (Z) |
| 21 | $(-CH_2-)_6$ | H | H | H | 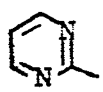 x 2 HCl | 160 (Z) |
| 22 | $(-CH_2-)_6$ | H | H | $CH_3$ | 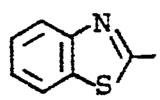 x 2 HCl | 135-137 |
| 23 | $(-CH_2-)_6$ | H | H | H | 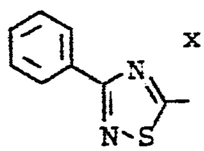 x 2 HCl | 155-157 (Z) |
| 24 | $(-CH_2-)_6$ | H | H | H | 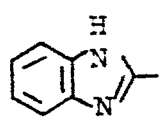 x 2 HCl | |

Anwendungsbeispiele

In dem nachfolgenden Beispiel wird die nachstehend angegebene Verbindung als Vergleichssubstanz eingesetzt:

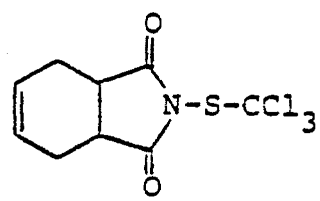

Beispiel

Botrytis-Test (Bohne) protektiv

Lösungsmittel: 4,7 Gewichtsteile Aceton
Emulgator: 0,3 Gewichtsteile Alkyl-aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test z.B. die Verbindungen gemäß folgender Herstellungsbeispiele: 1 , 16, 17, 18, 19, 20, 22, 23, 9, 4, 3 und 6.

Patentansprüche

1. Alkylen-bis-heterocyclyl-biguanide der allgemeinen Formel (I)

$$A-\underset{R^3}{\underset{|}{N}}-\overset{NH}{\overset{\|}{C}}-NH-\overset{NH}{\overset{\|}{C}}-\underset{R^1}{\underset{|}{N}}-R-\underset{R^2}{\underset{|}{N}}-\overset{NH}{\overset{\|}{C}}-NH-\overset{NH}{\overset{\|}{C}}-\underset{R^3}{\underset{|}{N}}-A \qquad (I)$$

in welcher

A für gegebenenfalls substituierte Heterocyclen steht,

R für Alkylen steht und

$R^1$, $R^2$ und $R^3$ gleich oder verschieden sind und für Wasserstoff, Alkyl oder gegebenenfalls substituiertes Aralkyl stehen und

deren Säureadditionssalze.

2. Alkylen-bis-heterocyclyl-biguanide der allgemeinen Formel (I) gemäß Anspruch 1, in welcher

A für einen gegebenenfalls einfach bis sechsfach, gleich oder verschieden substituierten 5- oder 6-gliedrigen, gesättigten oder gegebenenfalls teilweise oder vollständig gesättigten Heterocyclus mit 1 bis 4 gleichen

0127062

oder verschiedenen Heteroatomen steht, wobei der Heterocyclus gegebenenfalls benzoannelliert sein kann oder gegebenenfalls eine oder mehrere Ketogruppen im Heterocyclus enthalten sind. Als Substituenten des Heterocyclus seien genannt: Halogen, Alkyl mit 1 bis 18 Kohlenstoffatomen, Alkylthio, Alkoxy oder Alkylsulfonyl mit jeweils 1 bis 8 Kohlenstoffatomen im Alkylteil und gegebenenfalls einfach bis sechsfach, gleich oder verschieden durch Halogen, Alkyl mit 1 bis 4 Kohlenstoffatomen oder Halogenalkyl mit 1 bis 3 Kohlenstoffatomen und 1 bis 5 gleichen oder verschiedenen Halogenatomen substituiertes Phenyl, ferner Halogenalkoxy, Halogenalkylthio und Halogenalkylsulfonyl mit 1 bis 3 Kohlenstoffatomen und 1 bis 5 gleichen oder verschiedenen Halogenatomen im jeweiligen Halogenalkylteil, ferner Phenoxy, Phenylthio und Phenylsulfonyl, die gegebenenfalls im Phenylteil durch Halogen oder Alkyl mit 1 bis 4 Kohlenstoffatomen substituiert sein können. Der ankondensierte Benzolring kann gegebenenfalls einfach bis vierfach, gleich oder verschieden durch Halogen, Alkyl, Alkoxy oder Alkylthio mit jeweils 1 bis 4 Kohlenstoffatomen in dem jeweiligen Alkylteil substituiert sein.

R für geradkettiges oder verzweigtes Alkylen mit 2 bis 8 Kohlenstoffatomen steht und

$R^1$, $R^2$ und $R^3$ gleich oder verschieden sind und für Wasserstoff, Alkyl mit 1 bis 20 Kohlenstoffatomen oder für Aralkyl mit 6 bis 10 Kohlenstoffatomen im Aryl- und 1 bis 6 Kohlenstoffatomen im Alkylteil steht, wobei der Arylteil ein- bis fünffach, gleich oder verschieden durch Alkyl mit 1 bis 6 Kohlenstoffatomen und/oder Halogen substituiert sein kann.

3. Alkylen-bis-heterocyclyl-biguanide der allgemeinen Formel (I) gemäß Anspruch 1, wobei

A für einen gegebenenfalls ein- bis vierfach, gleich oder verschieden substituierten 5- oder 6-gliedrigen, gesättigten oder teilweise oder vollständig ungesättigten Heterocyclus mit 1 oder 2 Sauerstoff- und/oder Schwefel- und/oder 1 bis 4 Stickstoffatomen steht, der gegebenenfalls benzoannelliert ist und gegebenenfalls eine oder zwei Ketogruppen enthält. Als Substituenten der Heterocyclen kommen in Frage: Fluor, Chlor und Brom, Alkyl, Alkylthio, Alkoxy und Alkylsulfonyl mit jeweils 1 bis 6 Kohlenstoffatomen im Alkylteil, gegebenenfalls ein- bis vierfach, gleich oder verschieden durch Fluor, Chlor, Brom, Methyl, Ethyl, n-Propyl, iso-Propyl, Trichlormethyl, Trifluormethyl, Dichlorfluormethyl und Tetrachlorethyl substituiertes Phenyl ferner

Halogenalkoxy, Halogenalkylthio und Halogenalkylsulfonyl mit 1 oder 2 Kohlenstoffatomen und ein bis 5 Chlor- und/oder Fluoratomen, ferner gegebenenfalls ein- bis dreifach, gleich oder verschieden durch Fluor, Chlor, Brom, Methyl, Ethyl, n-Propyl und iso-Propyl substituiertes Phenoxy, Phenylthio oder Phenylsulfonyl. Der ankondensierte Benzolring kann gegebenenfalls ein- bis dreifach, gleich oder verschieden durch Chlor, Fluor, Methyl, Ethyl, n-Propyl, iso-Propyl, Methoxy, Ethoxy, n-Propoxy, iso-Propoxy, Methylthio, Ethylthio, n-Propylthio und/oder iso-Propylthio substituiert sein.

R für Ethylen, iso-Propylen, n-Propylen, n-Butylen, iso-Butylen, sec.-Butylen, Tetramethylen, Pentamethylen, Hexamethylen, Heptamethylen, Octamethylen, 2,5-Dimethylhexylen-, 1-Methylethylen, steht und

$R^1$,$R^2$ und $R^3$ gleich oder verschieden sind und für Wasserstoff, Alkyl mit 1 bis 10 Kohlenstoffatomen, für Phenethyl oder Benzyl steht, wobei der jeweilige Phenylring ein- bis dreifach, gleich oder verschieden durch Alkyl mit 1 bis 4 Kohlenstoffatomen und Halogen substituiert sein kann.

4. Alkylen-bis-heterocyclyl-biguanide der allgemeinen Formel (I) gemäß Anspruch 1, wobei

A für einen gegebenenfalls ein- bis dreifach, gleich oder verschieden substituierten Thienyl-, Benzothienyl-, Furyl-, Pyrrolyl-, Imidazolyl-, Pyrazolyl-, Pyridyl-, Pyrazinyl-, Pyrimidinyl-, Pyridazinyl-, Chinolyl, Phthalazinyl-, Chinoxalinyl-, Isoxazolyl-, Isothiazolyl-, Furazanyl-, Pyrrolidinyl-, Imidazolidinyl-, 1,2,3-Triazolidinyl-, 1,2,4-Triazolyl-, 1,2,3-Triazolyl-, 1,3,4-Triazolyl-, Tetrazolyl-, Thiazolyl-, Oxazolyl-, 1,2,4-Thiadiazolyl-, 1,2,5-Thiadiazolyl-, Benzthiazolyl-, Benzoxazolyl- oder Benzimidazolylrest steht, der eine Ketogruppe enthalten kann. Als Substituenten der Heterocyclen kommen in Frage: Chlor, Fluor, Methyl, Ethyl, iso-Propyl, Methylthio, Ethylthio, Methoxy, Ethoxy, Trifluormethyl, Trifluormethylthio, Phenyl, Chlorphenyl, Trifluormethyl-phenyl, Trifluormethylthio-phenyl, Phenoxy. Als Substituenten für den ankondensierten Benzolring seien genannt: Chlor, Fluor, Methyl, Ethyl, Methoxy und Methylthio.

R steht für Alkylen mit 6 Kohlenstoffatomen,

$R^1$ und $R^2$ für Wasserstoff und

$R^3$ für Wasserstoff oder Methyl stehen.

5. Verfahren zur Herstellung von Alkylen-bis-heterocyclyl-biguaniden der allgemeinen Formel (I)

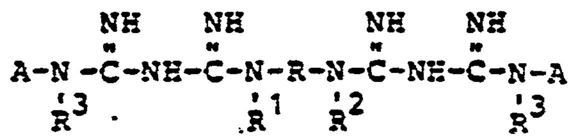

(I)

in welcher

A für gegebenenfalls substituierte Heterocyclen steht,

R für Alkylen steht und

$R^1$, $R^2$ und $R^3$ gleich oder verschieden sind und für Wasserstoff, Alkyl oder gegebenenfalls substituiertes Aralkyl stehen

und deren Säureadditionssalzen,

dadurch gekennzeichnet, daß man

Alkylen-bis-dicyandiamide der allgemeinen Formel (II)

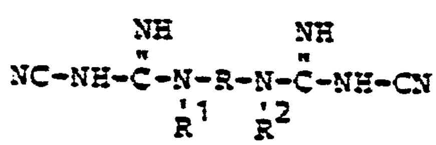

(II)

in welcher

$R, R^1$ und $R^2$ die oben angegebene Bedeutung haben,

mit Aminoheterocyclen der allgemeinen Formel (III)

$$\underset{R^3}{\text{A-NH}} \qquad \text{(III)}$$

in welcher

$R^3$ und A die oben angegebene Bedeutung haben,

als freie Verbindungen oder in Salzform, gegebenenfalls in einem Lösungs- oder Verdünnungsmittel bei Temperaturen zwischen 80 und 200°C umsetzt.

6. Schädlingsbekämpfungsmittel, gekennzeichnet durch einen Gehalt an mindestens einem Alkylen-bis-heterocyclyl-biguanid der Formel (I) gemäß Ansprüchen 1 und 5.

7. Verwendung von Alkylen-bis-heterocyclyl-biguaniden der Formel (I) gemäß Ansprüchen 1 und 5 zur Bekämpfung von Schädlingen.

8. Verfahren zur Bekämpfung von Schädlingen, dadurch gekennzeichnet, daß man Alkylen-bis-heterocyclyl-biguanide der Formel (I) gemäß Ansprüchen 1 und 5 auf Schädlinge und/oder ihren Lebensraum einwirken läßt.

9. Verfahren zur Herstellung von Schädlingsbekämpfungsmitteln, dadurch gekennzeichnet, daß man Alkylen-bis-heterocyclyl-biguanide der Formel (I) gemäß Ansprüchen 1 und 5 mit Streckmitteln und/oder oberflächenaktiven Mitteln vermischt.